(19) Europäisches Patentamt
European Patent Office
Office européen des brevets

(11) EP 0 530 136 B1

(12) EUROPÄISCHE PATENTSCHRIFT

(45) Veröffentlichungstag und Bekanntmachung des Hinweises auf die Patenterteilung:
**21.02.1996 Patentblatt 1996/08**

(51) Int. Cl.[6]: **A01N 43/80**, C07D 275/04

(21) Anmeldenummer: **92810521.2**

(22) Anmeldetag: **08.07.1992**

(54) **Nematizide Mittel**
Nematicidal agents
Agents nématicides

(84) Benannte Vertragsstaaten:
**CH DE ES FR GB IT LI NL**

(30) Priorität: **17.07.1991 CH 2118/91**

(43) Veröffentlichungstag der Anmeldung:
**03.03.1993 Patentblatt 1993/09**

(73) Patentinhaber: **CIBA-GEIGY AG**
**CH-4002 Basel (CH)**

(72) Erfinder: **Sutter, Marius, Dr.**
**CH-4102 Binningen (CH)**

(56) Entgegenhaltungen:
**EP-A- 0 367 365** **EP-A- 0 454 621**
**DE-A- 2 626 967**

- **J.CHEM.SOC.PERKIN TRANS. I 1983, Seiten 2973 - 2977 L.K.A.RAHMAN ET AL. '7-Substituted Benzo[b]thiophenes and 1,2-Benisothiazoles. Part 1.'**
- **ARZNEIMITTEL-FORSCHUNG Bd. 30, Nr. 11, 1980, Seiten 1831 - 1838 A.FRANKE ET AL. 'Neue â-Symphatolytika: Synthesen und pharmakologische Wirkung isomerer Benzthiazol- und Benzoxazol-Derivate'**
- **TETRAHEDRON Bd. 21, 1965, Seiten 3969 - 3980 D.S.KEMP ET AL. 'The N-Ethylbenzisoxazolium Cation - I'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 530 136 B1

Printed by Rank Xerox (UK) Business Services
2.9.9/3.4

## Beschreibung

Die vorliegende Erfindung betrifft neue nematizide Mittel, die als Wirkstoff mindestens ein Hydrocarbyloxy-benzisothiazol der allgemeinen Formel I enthalten und ihre Verwendung zur Bekämpfung von Nematoden, insbesondere von pflanzenschädigenden Nematoden.

Die Erfindung betrifft ferner neue, nematizid wirksame Hydrocarbyloxy-benzisothiazole im Umfang der Formel I und Verfahren zu ihrer Herstellung.

Die Benzisothiazole gemäss der vorliegenden Erfindung entsprechen der allgemeinen Formel I

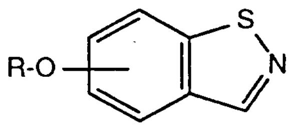

(I),

worin die Gruppe RO- in einer der Positionen des Benzolrings steht und R einen reinen oder halogenierten Kohlenwasserstoffrest bedeutet, der aliphatisch oder cycloaliphatisch, gesättigt oder ungesättigt ist.

Die als Nematizide bekannten Verbindungen haben bisher die in der Praxis an sie gestellten Ansprüche nicht befriedigen können.

Ziel der vorliegenden Erfindung ist es daher, neue nematizide Mittel mit vorteilhaften Eigenschaften bereitzustellen.

Durch die Bereitstellung der erfindungsgemässen Mittel, welche als Wirkstoff die Verbindungen der Formel I und zusätzlich mindestens noch einen Träger enthalten, ist es nun gelungen, einen wertvollen Beitrag zur Bekämpfung der beträchtliche landwirtschaftliche Schäden an Pflanzgut verursachenden Pflanzennematoden zu leisten. Auf diese Weise können Ernteeinbussen bei Kulturpflanzen eingedämmt werden. Zu nennen wären insbesondere Kartoffeln, Getreide, Rüben, Raps, Kohl, Tabak, Sojabohnen, Baumwolle, Mais, Reis und Gemüse sowie Pflanzenbestand in Baumschulen und im Zierpflanzenbau. Die erfindungsgemässen Mittel zeichnen sich dabei insbesondere in der wirkungsvollen Bekämpfung von wurzelparasitären Bodennematoden wie z.B. solchen der Gattungen Heterodera und Globodera (zystenbildende Nematoden), Meloidogyne (Wurzelgallennematoden) sowie der Gattungen Radopholus, Pratylenchus, Tylenchulus, Longidorus, Trichodorus und Xiphinema aus. Ferner lassen sich mit den erfindungsgemässen Mitteln die Nematodengattungen Ditylenchus (Stengelparasiten) Aphelenchoides (Blattnematoden) und Anguina (Blütennematoden) wirkungsvoll bekämpfen.

Mit den Mitteln, welche als Wirkstoff die Verbindungen der Formel I enthalten, lassen sich vorzugsweise besonders schädliche Nematodenarten der Gattung Meloidogyne, wie z.B. Meloidogyne incognita, sowie der Gattung Heterodera, wie z.B. Heterodera glycines (Sojabohnen-Zystennematode) erfolgreich bekämpfen.

Zur Bekämpfung der Pflanzennematoden und zur Gesunderhaltung des Pflanzgutes können die neuen Wirkstoffe kurativ, präventiv oder systemisch eingesetzt werden. Dabei entfalten sie eine breit gefächerte Aktivität gegen die verschiedenen Nematodenspecies und werden somit den Erfordernissen der Praxis gerecht.

In der EP-A-367,365 werden Benzisothiazol-Derivate der Formel

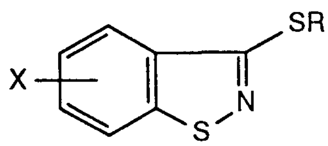

als Nematizide vorgeschlagen, wobei ein halogenierter Kohlenwasserstoffrest R, der in 3-Stellung über eine Schwefelbrücke gebunden ist, das offenkundig für die Wirkung essentielle Strukturelement darstellt. Es konnte aus dieser Referenz kein Hinweis abgeleitet werden, dass das Fehlen dieses Elements zu nematizid weit wirksameren Benzisothiazol-Derivaten führen würde.

Ueberraschenderweise besitzen nämlich die Verbindungen der Formel I keine oder nur eine sehr geringe Phytotoxizität, vor allem aber eine sehr günstige, akute Warmblüter-Toxizität, die bis zu fünfzigfach geringer ist als die Toxizität bekannter Phosphorsäureester- und Carbamat-Nematizide. Diese beiden Eigenschaften heben die vorliegenden Wirkstoffe der Formel I aus der Vielzahl der Präparate heraus, die bisher als Pflanzen-Nematizide vorgeschlagen wurden.

Die vorliegende Erfindung stellt daher eine ganz ungewöhnliche Bereicherung der Technik dar.

Die vor- und nachstehend verwendeten Allgemeinbegriffe haben, sofern nicht abweichend definiert, die nachfolgend aufgeführte Bedeutung:

Halogen ist Fluor, Chlor, Brom oder Iod, vor allem Fluor, Chlor oder Brom, insbesondere Fluor.

Kohlenwasserstoffreste können 1 bis 4 C-Atome enthalten, bevorzugt 1-3 C-Atome und können, je nach Anzahl der C-Atome, geradkettig, wie Methyl, Ethyl, Propyl oder Butyl, oder verzweigt, wie Isopropyl, Isobutyl, sec.-Butyl oder tert.-Butyl, sein.

Cycloaliphatische Kohlenwasserstoffreste sind z.B. Cyclopropyl, Methylcyclopropyl, Cyclopropylmethyl oder Cyclobutyl.

Ungesättigte Kohlenwasserstoffreste enthalten mindestens eine Doppelbindung und/oder eine Dreifachbindung und können, je nach Anzahl der C-Atome, geradkettig, wie z.B. Vinyl, Allyl, Butenyl, Propinyl, Butinyl, oder verzweigt, wie z.B.Methylallyl, sein.

Halogenierte Kohlenwasserstoffreste können gesättigt oder ungesättigt, aliphatisch oder cycloaliphatisch, teilweise oder perhalogeniert sein. Beispiele sind Fluormethyl, Difluormethyl, Trifluormethyl, 1,2-Difluorethyl, Difluorbutenyl, Chlorallyl, Dichlorvinyl, Bromvinyl, Brompropinyl, 1-Fluorcyclopropyl.

Der Substituent RO- kann in jeder der vier Stellungen 4, 5, 6 oder 7 des Benzolteils im Molekül stehen. Eine der bevorzugten Stellungen ist die zum S-Atom nachbarständige 7-Position.

7-Methoxybenzisothiazol ist aus J. Chem. Soc. Perkin Trans I (1983), pp. 2973-2977 bekannt. Ein technischer Verwendungszweck ist dort nicht beschrieben worden.

4-Methoxybenzisothiazol wurde in J.Chem.Soc. C) 3994-3999 (1971) beschrieben, ohne Angabe eines technischen Verwendungszweckes.

Bevorzugt sind im Rahmen dieser Erfindung Mittel, in denen die aktive Komponente eine Verbindung der Formel I ist, wobei R einen gegebenenfalls halogenierten Kohlenwasserstoffrest mit bis zu 4 C-Atomen darstellt.

Besonders bevorzugt sind Mittel, die als aktive Komponente eine Verbindung der Formel I'

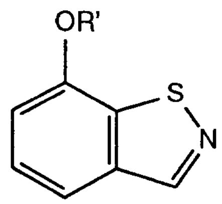

(I')

enthalten, worin R' einen gegebenenfalls halogenierten Kohlenwasserstoff mit 1 bis 3 C-Atomen bedeutet.

Eine weitere bevorzugte Gruppe von Mitteln enthält als aktive Komponente eine Verbindung ausgewählt aus 7-Methoxybenzisothiazol, 7-Difluormethoxy-benzisothiazol, 7-Allyloxybenzisothiazol, 7-Propargyloxy-benzisothiazol, 4-Methoxy-benzisothiazol, 5-Methoxy-benzisothiazol, 6-Methoxy-benzisothiazol.

Weiterhin bevorzugte Mittel enthalten als aktive Komponente 6-Ethoxy-benzisothiazol und 6-Allyloxy-benzisothiazol.

Ein ganz besonders bevorzugtes Mittel enthält als aktive Komponente 7-Methoxy-benzisothiazol.

Ein weiterer Gegenstand der vorliegenden Erfindung sind Hydrocarbyloxy-benzisothiazole der Formel I"

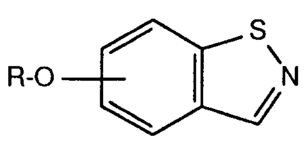

(I"),

worin die Gruppe RO- in einer der Positionen des Benzolrings steht und R einen reinen oder halogenierten Kohlenwasserstoffrest bedeutet, der aliphatisch oder cycloaliphatisch, gesättigt oder ungesättigt ist, mit der Massgabe, dass eine in 4- oder 7-Stellung befindliche Methoxygruppe halogeniert ist.
Bevorzugt sind 6-Ethoxy-benzisothiazol und 6-Allyloxy-benzisothiazol.

Die Erfindung betrifft ferner ein Verfahren zur Herstellung der Verbindungen der Formel I, dadurch gekennzeichnet, dass man einen substituierten Halo-benzaldehyd der Formel II mit einem Thiol $R_8SH$ in Lösung, in Gegenwart einer Base zum Thioether der Formel III umsetzt und letzteren, mit oder ohne Isolierung, mit Hydroxylamin-O-sulfonsäure in Lösung umsetzt und zum erwünschten Produkt zyklisiert,

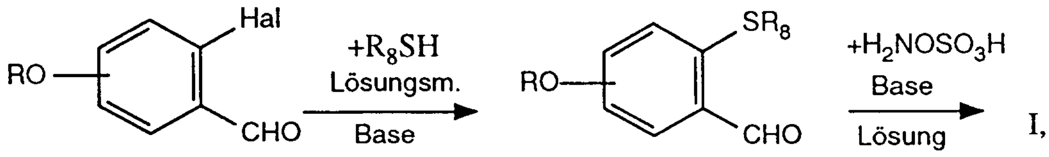


II III

wobei Hal Halogen und $R_8$ $C_1$-$C_{30}$-Alkyl, $C_3$-$C_7$-Cycloalkyl oder Benzyl bedeuten.

Als Base für die erste Stufe dieser Reaktionsfolge eignen sich beispielsweise Hydride, Amide, Alkoholate, Carbonate oder Hydrogencarbonate von Alkali- oder Erdalkalimetallen, vorzugsweise Kalium- oder Natriumcarbonat. Vorteilhaft ist die Reaktion in Alkoholen oder in aprotischen Lösungsmitteln, wie z.B. Dimethylformamid, Dimethylsulfoxid, Dimethylpropylenharnstoff oder Dimethylethylenharnstoff oder in Äthern, wie Diethyläther oder Tetrahydrofuran bei 0-70°C, vorzugsweise bei Raumtemperatur durchzuführen.

Die Reaktion kann auch in Gegenwart von Metallsalzen wie z.B. Kupfer, Nickel oder Palladiumsalze durchgeführt werden.

Als Base für die zweite Stufe eignen sich Alkalicarbonate- oder -hydrogencarbonate, beispielsweise Natriumhydrogencarbonat; ferner tert. Amine, beispielsweise Triethylamin oder Pyridin. Für die zweite Stufe eignen sich aprotische organische Lösungsmittel, wie beispielsweise Methylenchlorid; Ether, wie beispielsweise Diethylether, Dioxan oder Tetrahydrofuran; Ester, wie beispielsweise Ethylacetat, Kohlenwasserstoffe, wie beispielsweise Hexan, Cyclohexan, Methylcyclohexan oder Toluol und deren Mischungen mit Wasser. Die Reaktionstemperatur der zweiten Stufe liegt zwischen -20 und 100°C, vorzugsweise bei 20 bis 50°C.

Eine bevorzugte Ausführungsform des Verfahrens ist dadurch gekennzeichnet, dass man den Thioether der Formel III mit Hydroxylamin-O-sulfonsäure in Lösung umsetzt und durch Zugabe einer Base zum erwünschten Produkt zyklisiert.

Eine weitere Ausführungsform zur Herstellung von Verbindungen der Formel Ib

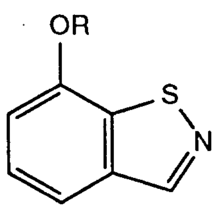

(Ib),

besteht darin, dass man einen Benzaldehyd der Formel IV mit Benzylmercaptan in Lösung, in Gegenwart einer Base zum Thioether der Formel V umsetzt und letzteren, mit oder ohne Isolierung, mit Hydroxylamin-O-sulfonsäure in Lösung umsetzt und zum erwünschten Produkt zyklisiert,

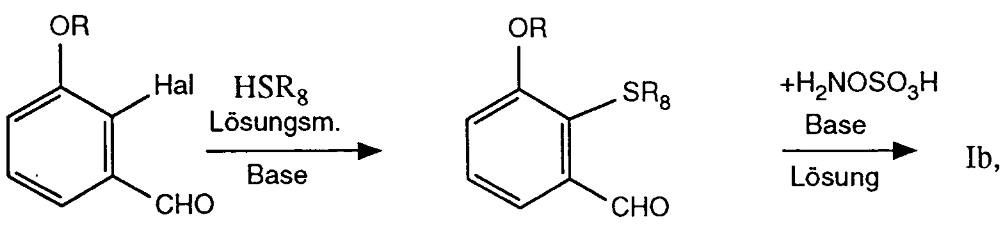


IV V

wobei Hal Halogen und $R_8$ Benzyl bedeuten.

Eine bevorzugte Ausführungsform ist dadurch gekennzeichnet, dass man den Thioether der Formel V mit Hydroxylamin-O-sulfonsäure in Lösung umsetzt und zum erwünschten Produkt zyklisiert; ganz besonders bevorzugt wird das

Zwischenprodukt der Formel V mit Hydroxylamin-O-sulfonsäure bei pH = 4-6 umgesetzt und die Zyklisierung durch anschliessende Base-Zugabe durchgeführt.

Eine weitere Ausführungsform zur Herstellung von Verbindungen der Formel I besteht darin, dass man ein Benzaldehyd-Derivat der Formel VI mit Hydroxylamin oder einem Salz davon zum entsprechenden Oxim der Formel VII umsetzt und dieses mit einer starken wasserbindenden Säure zyklisiert, wobei R die für Formel I angegebene Bedeutung hat und $R_8$ $C_1$-$C_{30}$-Alkyl, $C_3$-$C_7$-Cycloalkyl oder Benzyl bedeuten.

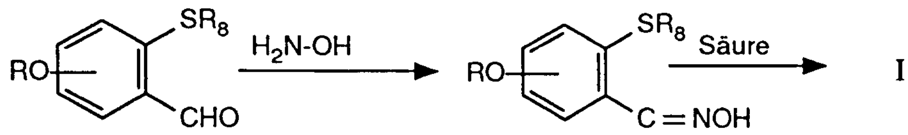


VI VII

Bevorzugt wird für die Zyklisierung als starke Säure Polyphosphorsäure oder Phosphorpentoxid in Methansulfonsäure verwendet; ganz besonders bevorzugt wird die Reaktion in einem Gemisch von 1-20 Gewichtsprozent Phosphorpentoxid in Methansulfonsäure bei -10° bis +100°C durchgeführt.

Verbindungen der Formel I können weiterhin hergestellt werden, indem ein phenolartiges Hydroxy-benzisothiazol der Formel VIII

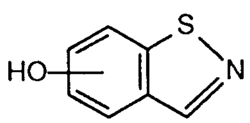

(VIII)

mit einem Reagenz R-X umgesetzt wird, in dem R die für Formel I gegebene Bedeutung hat und X Halogen, Tosylat, oder Mesylat bedeutet.

Die noch neuen Zwischenprodukte der Formel III stellen einen weiteren Gegenstand vorliegender Erfindung dar.

Von den Verbindungen der Formel III sind als Zwischenprodukte jene zur Synthese hervorzuheben, bei welchen $R_8$ $C_1$-$C_6$-Alkyl oder den Benzylrest bedeutet.

Die Ausgangsprodukte der erwähnten Herstellungsverfahren sind entweder bekannte, im Handel erhältliche Verbindungen oder können nach bekannten Verfahren hergestellt werden.

Die noch neuen Zwischenprodukte der Formel VIII, 6-Hydroxybenzisothiazol und 7-Hydroxybenzisothiazol, stellen einen weiteren Gegenstand vorliegender Erfindung dar.

Sie lassen sich prinzipiell nach den vorgängig beschriebenen Methoden herstellen.

Darüber hinaus schliesst die vorliegende Erfindung die Herstellung nematizider Mittel ein, die gekennzeichnet ist durch das innige Vermischen von Aktivsubstanzen der Formel I mit einer oder mehreren hierin beschriebenen Trägerstoffen und Hilfsingredienzen. Die erfindungsgemässen Mittel enthalten dementsprechend eine wirksame Menge mindestens einer der Verbindungen der Formel I.

Die Erfindung umfasst auch ein Verfahren zur Behandlung von Pflanzen, indem man eine Verbindung der Formel I oder ein Mittel, dass mindestens eine Verbindung der Formel I enthält, auf die Pflanze, auf Pflanzenteile oder auf den Nährboden für die Pflanze appliziert. Bevorzugte Verbindungen sind
7-Methoxy-benzisothiazol, 7-Difluormethoxy-benzisothiazol, 7-Allyloxy-benzisothiazol, 7-Propargyloxy-benzisothiazol, 4-Methoxy-benzisothiazol, 5-Methoxy-benzisothiazol, 6-Methoxy-benzisothiazol; besonders bevorzugt ist 7-Methoxy-benzisothiazol. Weiterhin bevorzugte Verbindungen sind 6-Ethoxy-benzisothiazol und 6-Allyloxy-benzisothiazol.

Die Verbindungen der Formel I können aber auch auf Samenkörner aufgebracht werden (Beizung/Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel, Knospen oder Blätter.

Die Verbindungen der Formel I werden in unveränderter Form oder vorzugsweise zusammen mit den in der Formulierungstechnik üblichen Hilfsmitteln eingesetzt. Sie werden z.B. zu Emulsionskonzentraten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Verstäuben, Verstreuen oder Giessen werden gleich wie die Art der Mittel den angestrebten Zielen und den gegebenen Verhältnissen entsprechend gewählt. Günstige Aufwandmengen liegen im allgemeinen bei 0,1 bis 10 kg

Aktivsubstanz (AS) je ha; bevorzugt 0,3 bis 5 kg AS/ha. Bei der Verwendung als Saatbeizmittel werden mit Vorteil Dosierungen von 0,001 bis 2 g AS pro kg Saatgut angewendet.

Wirkstoffe der Formel I werden üblicherweise in Form von formulierten Zusammensetzungen verwendet und können gleichzeitig oder nacheinander mit weiteren Wirkstoffen auf die zu behandelnde Fläche oder Pflanze gegeben werden. Diese weiteren Wirkstoffe können auch andere in der Landwirtschaft angewendete Mittel umfassen, die in ihrer Nutzanwendung der Produktionssteigerung durch Förderung des Nutzpflanzenwachstums dienen, wie Düngemittel, Herbizide, Insektizide, Fungizide, Molluskizide u.a., oder Gemische mehrerer dieser Präparate sein, zusammen mit gegebenenfalls weiteren in der Formulierungstechnik üblichen Lösungsmitteln, Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Substanzen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$)genannt, wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die neuen, in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien.

Die agrochemischen Zubereitungen enthalten eine wirksame Menge, d.h. in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

H. <u>Herstellungsbeispiele</u>:

<u>H.1 Herstellung von 7-Methoxy-benzisothiazol [Verfahren 1] [Verb. Nr. 1.1].</u>

136,5 g 2-Chlor-3-methoxy-benzaldehyd werden mit 136,5 g Kaliumcarbonat in 800 ml Dimethylformamid verrührt. Zu dieser Suspension wird 90,6 ml Benzylmercaptan getropft und das Gemisch über Nacht bei Raumtemperatur und anschliessend einen Tag lang bei 100°C gerührt. Das Reaktionsgemisch wird auf Wasser gegossen und mit Diethylether extrahiert. Die organische Phase wird über Natriumsulfat getrocknet, filtriert und eingeengt. Das erhaltene Oel wird in 500 ml Wasser (auf pH = 5 gepuffert) vorgelegt und unter Eiskühlung mit 110.7 g Hydroxylamin-O-sulfonsäure versetzt. Nach 1 Stunde Rühren bei Raumtemperatur wird 300 ml Wasser und 300 ml Methylenchlorid zugegeben und das Ganze portionenweise mit 276,6 g Natriumhydrogencarbonat versetzt. Nach 2 Stunden Rührenlassen bei Raumtemperatur wird das Reaktionsgemisch mit Methylenchlorid extrahiert, die organische Phase wird mit konzentrierter NaCl-Lösung (Sole) gewaschen, über Natriumsulfat getrocknet, filtriert und eingeengt. Das erhaltene Oel liefert nach chromatographischer Reinigung 7-Methoxybenzisothiazol, $n_D^{20}$ = 1,6190.

<u>H.2 Herstellung von 7-Methoxy-benzisothiazol [Verfahren 2].</u>

5 g 2-Benzylthio-3-methoxy-benzaldehyd) wird in 40 ml Ethanol gelöst und mit 3,9 g Hydroxylamin-hydrochlorid versetzt. Es werden zu dieser Lösung 40 ml Acetatpuffer (pH = 5) zugegeben und 30 Minuten bei Raumtemperatur gerührt. Das Gemisch wird auf Wasser gegossen und mit Essigsäureethylester extrahiert. Die organische Phase wird mit Sole gewaschen, über Natriumsulfat getrocknet, filtriert und eingeengt. Das erhaltene Oel wird zu einer Suspension aus 1,6 g Phosphorpentoxid in 20 ml Methansulfonsäure gegeben und bei Raumtemperatur über Nacht gerührt. Das Reaktionsgemisch wird auf Eis gegossen und mit Essigsäureethylester extrahiert. Die organische Phase wird mit gesättigter Natriumhydrogencarbonatlösung und Sole gewaschen, über Natriumsulfat getrocknet, filtriert und eingeengt. Nach chromatographischer Reinigung erhält man 7-Methoxybenzisothiazol als Oel.

<u>H.3 Herstellung von 7-Hydroxybenzisothiazol (Zwischenprodukt)</u>

174 g 2-Chlor-3-hydroxy-benzaldehyd wird in 1 l Dimethylformamid gelöst, mit 126 ml Benzylmercaptan und 174 g Kaliumcarbonat versetzt und 2 Tage bei 100°C gerührt. Das Reaktionsgemisch wird auf Eiswasser gegossen, mit Diethylether extrahiert, die organische Phase mit Sole gewaschen, über Natriumsulfat getrocknet, filtriert und eingeengt.

Die Chromatographie an Kieselgel, mit Essigsäureethylester/Hexan (1:4) als Laufmittel ergibt 51,6 g 2-Benzylthio-3-hydroxy-benzaldehyd. Davon werden 31,6 g in 66 ml Wasser emulgiert und mit 16,1 g Hydroxylamin-O-sulfonsäure und 1,84 g Natriumsulfat versetzt. Nach einer Stunde werden je 32 ml Wasser und Methylenchlorid zugegeben und das Gemisch wird portionenweise mit 40,4 g Natriumhydrogencarbonat versetzt. Nach 2 Stunden Rührenlassen bei Raumtemperatur wird das Reaktionsgemisch in Methylenchlorid aufgenommen und die organische Phase mit 1N Kalilauge extrahiert. Die wässrig-basische Phase wird mit konzentrierter Chlorwasserstoffsäure auf pH 1 gestellt und mit Methylenchlorid extrahiert. Die organische Phase wird über Natriumsulfat getrocknet, filtriert und eingeengt. Umkristallisation aus Toluol liefert 5,93 g 7-Hydroxy-1,2-benzisothiazol vom Smp. 185-187°C.

<u>H.4 Herstellung von 7-Allyloxy-benzisothiazol [Verbindung Nr. 1.9]</u>

2,0 g 7-Hydroxy-1,2-benzisothiazol werden in 20 ml THF gelöst und bei 0°C mit 460 mg Natriumhydrid (60%ig) versetzt. Nach beendeter Gasentwicklung wird 1 ml Allylbromid zugetropft und das Reaktionsgemisch einen Tag lang bei Raumtemperatur gerührt. Es wird erneut 1 ml Allylbromid und 40 mg Natriumhydrid zugegeben und 2 Tage unter Rückfluss gerührt. Das Reaktionsgemisch wird auf Wasser gegossen, mit 1N Kalilauge basisch gestellt und mit Essigsäureethylester extrahiert. Die organische Phase wird mit Sole gewaschen, über Natriumsulfat getrocknet, filtriert und eingeengt. Chromatographie an Kieselgel mit Essigsäureethylester/Hexan (1:5) als Laufmittel ergibt 2,0 g 7-Allyloxy-1,2-benzisothiazol; $n_D^{20}$ = 1,6039

H.5 Herstellung von 7-Difluormethoxy-1,2-benzisothiazol [Verb. Nr. 1.2]

2,0 g 7-Hydroxy-1,2-benzisothiazol werden in 40 ml Acetonitril gelöst und mit 10,8 g festem Kaliumcarbonat versetzt. Bei 70°C wird während 18 h Freon 22 eingeleitet. Nach beendeter Gaseinleitung wird filtriert und das Filtrat eingeengt. Chromatographie an Kieselgel mit Essigsäureethylester/Hexan (4:1) als Laufmittel liefert 370 mg 7-Difiuormethoxy-1,2-benzisothiazol; Smp. 58-59°C.

Auf analoge Art lassen sich folgende 7-substituierte Benzisothiazole herstellen:

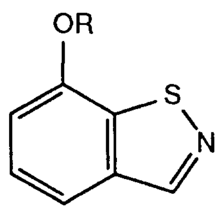

| Verb. Nr. | R | Physikal. Daten |
|---|---|---|
| 1.1 | $-CH_3$ | $n_D^{20}$ = 1,6190 |
| 1.2 | $-CHF_2$ | Smp. 58-59°C |
| 1.3 | $-CH_2F$ | |
| 1.4 | $-C_2H_5$ | $n_D^{20}$ = 1,5923 |
| 1.5 | $-CHF-CH_2F$ | |
| 1.6 | $-CH=CH_2$ | |
| 1.7 | $-CH_2-C\equiv CH$ | Smp. 74-76°C |
| 1.8 | $-CH_2-C\equiv CBr$ | |
| 1.9 | $-CH_2-CH=CH_2$ | $n_D^{20}$ = 1,6039 |
| 1.10 | $-CH_2-C(CH_3)=CH_2$ | |
| 1.11 | Cyclopropyl | |
| 1.12 | 1-Fluorocyclopropyl | |
| 1.13 | $-C\equiv CH$ | |
| 1.14 | Isopropyl | $n_D^{20}$ = 1,5680 |
| 1.15 | tert. Butyl | $n_D^{20}$ = 1,5669 |
| 1.16 | $-CF_3$ | $R_f$= 0,60 [Hexan/Ethylacetat 1:1] |
| 1.17 | $-CH=CHBr$ | $n_D^{20}$ = 1,3005 |
| 1.18 | $-CH_2CH_2CF=CF_2$ | $n_D^{20}$ = 1,5025 |
| 1.19 | $-CH_2CH_2CH_3$ | $n_D^{20}$ = 1,5815 |
| 1.20 | Cyclopropylmethyl | n= 1,5902 |
| 1.21 | $-CH=CCl_2$ | Smp. 73-74°C |

Auf analoge Art lassen sich folgende 4-substituierte Benzisothiazole herstellen:

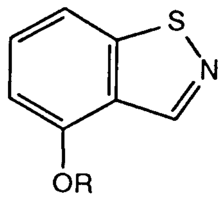

| Verb. Nr. | R | Physikal. Daten |
|---|---|---|
| 2.1 | $-CH_3$ | Smp. 70-71°C |
| 2.2 | $-CHF_2$ | |
| 2.3 | $-CH_2F$ | |
| 2.4 | $-C_2H_5$ | |
| 2.5 | $-CHF-CH_2F$ | |
| 2.6 | $-CH=CH_2$ | |
| 2.7 | $-CH_2-C\equiv CH$ | |
| 2.8 | $-CH_2-C\equiv CBr$ | |
| 2.9 | $-CH_2-CH=CH_2$ | |
| 2.10 | $-CH_2-C(CH_3)=CH_2$ | |
| 2.11 | Cyclopropyl | |
| 2.12 | 1-Fluorocyclopropyl | |
| 2.13 | $-C\equiv CH$ | |
| 2.14 | Isopropyl | |
| 2.15 | tert. Butyl | |
| 2.16 | $-CF_3$ | |

Auf analoge Art lassen sich folgende 5-substituierte Benzisothiazole herstellen:

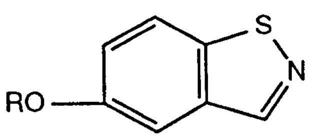

| Verb. Nr. | R | Physikal. Daten |
|---|---|---|
| 3.1 | $-CH_3$ | $n_D^{20} = 1,6021$ |
| 3.2 | $-CHF_2$ | |
| 3.3 | $-CH_2F$ | |
| 3.4 | $-C_2H_5$ | |
| 3.5 | $-CHF-CH_2F$ | |
| 3.6 | $-CH=CH_2$ | |
| 3.7 | $-CH_2-C\equiv CH$ | |
| 3.8 | $-CH_2-C\equiv CBr$ | |
| 3.9 | $-CH_2-CH=CH_2$ | |
| 3.10 | $-CH_2-C(CH_3)=CH_2$ | |
| 3.11 | Cyclopropyl | |
| 3.12 | 1-Fluorocyclopropyl | |
| 3.13 | $-C\equiv CH$ | |
| 3.14 | Isopropyl | |
| 3.15 | tert. Butyl | |
| 3.16 | $-CF_3$ | |

Auf analoge Art lassen sich folgende 6-substituierte Benzisothiazole herstellen:

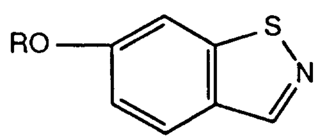

| Verb. Nr. | R | Physikal. Daten |
|---|---|---|
| 4.1 | $-CH_3$ | $n_D^{20} = 1{,}6235$ |
| 4.2 | $-CHF_2$ | |
| 4.3 | $-CH_2F$ | |
| 4.4 | $-C_2H_5$ | Smp. 48-50°C |
| 4.5 | $-CHF-CH_2F$ | |
| 4.6 | $-CH=CH_2$ | |
| 4.7 | $-CH_2-C\equiv CH$ | |
| 4.8 | $-CH_2-C\equiv CBr$ | |
| 4.9 | $-CH_2-CH=CH_2$ | $n_D^{20} = 1{,}6093$ |
| 4.10 | $-CH_2-C(CH_3)=CH_2$ | |
| 4.11 | Cyclopropyl | |
| 4.12 | 1-Fluorocyclopropyl | |
| 4.13 | $-C\equiv CH$ | |
| 4.14 | Isopropyl | |
| 4.15 | tert. Butyl | |
| 4.16 | $-CF_3$ | |

F. Formulierungsbeispiele für flüssige Wirkstoffe der Formel I (% = Gewichtsprozent)

F.1 Emulsions-Konzentrate

| | a) | b) | c) |
|---|---|---|---|
| Wirkstoff aus den Tabellen 1-4 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykolether (30 Mol Ethylenoxid) | - | - | 12 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

F.2 Lösungen

| | a) | b) | c) | d) |
|---|---|---|---|---|
| Wirkstoff aus den Tabellen 1-4 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |
| (MG = Molekulargewicht) | | | | |

Die Lösungen sind zur Anwendung in Form kleinster Tropfen geeignet.

F.3 Granulate

| | a) | b) |
|---|---|---|
| Wirkstoff aus den Tabellen 1-4 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

F.4 Stäubemittel

| | a) | b) |
|---|---|---|
| Wirkstoff aus den Tabellen 1-4 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

F.5 Spritzpulver

| | a) | b) | c) |
|---|---|---|---|
| Wirkstoff aus den Tabellen 1-4 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F.6 Emulsions-Konzentrat

| | |
|---|---|
| Wirkstoff aus den Tabellen 1-4 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

F.7 Stäubemittel

| | a) | b) |
|---|---|---|
| Wirkstoff aus den Tabellen 1-4 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

F.8 Extruder Granulat

| | |
|---|---|
| Wirkstoff aus den Tabellen 1-4 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

F.9 Umhüllungs-Granulat

| | |
|---|---|
| Wirkstoff aus den Tabellen 1-4 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

B. Biologische Beispiele

B.1 Wirkung gegen Meloidogyne incognita auf Tomaten

Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der formulierte Wirkstoff mittels Drenchapplikation in die Töpfe hineingegeben (0,0006 % Aktivsubstanz bezogen auf das Bodenvolumen). Die eingetopften Pflanzen werden nun bei einer Temperatur von 26 ± 1°C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgallen-Index ("Root-Knot Index").

Unbehandelte jedoch infizierte Kontrollpflanzen weisen starke Wurzelgallbildung auf (= 100 %). Eine gute Wirkung zeigen dagegen die Verbindungen der Formel I mit weniger als 20 % Restbefall. Die Verbindungen Nr. 1.1, 1.2, 1.9, 2.1, 3.1, 4.1, 4.4, 4.9 und andere hemmen im obigen Versuch die Wurzelgallbildung sogar fast vollständig (0-10 % Restbefall).

B.2 Wirkung gegen Heterodera glycines auf Sojabohnen

Sandige Erde wird mit Eiern des Sojabohnenzystennematoden *H. glycines* infestiert, etwa 6000 Eier pro Topf. Anschliessend werden die zu prüfenden Substanzen in den entsprechenden Konzentrationen eingemischt. Die behandelte und infestierte Erde wird dann in 1c Töpfe (180 ccm) gefüllt und jeweils drei Sojabohen (cv. Maple Arrow) eingesät. Jede Behandlung wird dreimal wiederholt. Die Töpfe werden bei ca 27°C für vier bis fünf Wochen im Gewächshaus inkubiert. Die Pflanzen werden dann vorsichtig den Töpfen entnommen, die Wurzeln gewaschen und die Anzahl der Zysten bestimmt.

Die Verbindungen aus den Tabellen 1-4 zeigen gegen Heterodera glycines eine gute Wirkung, was sich in der fast vollständigen Reduktion der Zystenbildung zeigt. Insbesondere sind die Verbindungen 2.1, 3.1, 4.1, 4.4 und 4.9 zu nennen.

**Patentansprüche**

1. Schädlingsbekämpfungsmittel zur Bekämpfung oder Verhütung des Befalls von Pflanzen durch Nematoden, dadurch gekennzeichnet, dass dieses neben einem inerten Trägermaterial mindestens eine Verbindung der Formel

I

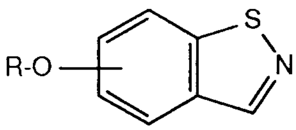

(I),

enthält, worin die Gruppe RO- in einer der Positionen des Benzolrings steht und R einen reinen oder halogenierten Kohlenwasserstoffrest bedeutet, der aliphatisch oder cycloaliphatisch, gesättigt oder ungesättigt ist, mit Ausnahme der Verbindungen 4-Methoxy-benzisothiazol und 7-Methoxy-benzisothiazol.

**2.** Mittel gemäss Anspruch 1, dadurch gekennzeichnet, dass R einen gegebenenfalls halogenierten Kohlenwasserstoffrest mit bis zu 4 C-Atomen darstellt.

**3.** Mittel gemäss Anspruch 2, dadurch gekennzeichnet, dass es als aktive Komponente eine Verbindung der Formel I'

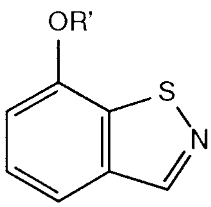

(I')

enthält, worin R' einen gegebenenfalls halogenierten Kohlenwasserstoffrest mit 1 bis 3 C-Atomen bedeutet.

**4.** Mittel gemäss Anspruch 1, dadurch gekennzeichnet, dass es als aktive Komponente eine Verbindung ausgewählt aus
7-Difluormethoxy-benzisothiazol,
7-Allyloxy-benzisothiazol
7-Propargyloxy-benzisothiazol,
5-Methoxy-benzisothiazol und
6-Methoxy-benzisothiazol enthält.

**5.** Mittel gemäss Anspruch 1, dadurch gekennzeichnet, dass es als aktive Komponente eine Verbindung ausgewählt aus
6-Ethoxy-benzisothiazol und 6-Allyloxy-benzisothiazol enthält.

**6.** Hydrocarbyloxy-benzisothiazol der Formel I"

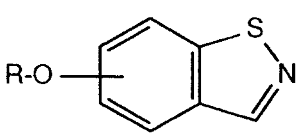

(I"),

worin die Gruppe RO- in einer der Positionen des Benzolrings steht und R einen reinen oder halogenierten Kohlenwasserstoffrest bedeutet, der aliphatisch oder cycloaliphatisch, gesättigt oder ungesättigt ist, mit der Massgabe, dass eine in 4- oder 7-Stellung befindliche Methoxygruppe halogeniert ist.

**7.** 6-Ethoxy-benzisothiazol und 6-Allyloxy-benzisothiazol.

8. Verfahren zur Herstellung einer Verbindung der Formel I

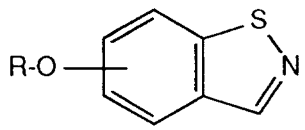

(I),

worin R die im Anspruch 1 gegebene Bedeutung hat, mit Ausnahme der Verbindungen 4-Methoxy-benzisothiazol und 7-Methoxy-benzisothiazol, dadurch gekennzeichnet, dass man einen substituierten Halo-benzaldehyd der Formel II mit einem Thiol $R_8SH$ in Lösung, in Gegenwart einer Base zum Thioether der Formel III umsetzt und letzteren, mit oder ohne Isolierung, mit Hydroxylamin-O-sulfonsäure in Lösung umsetzt und zum erwünschten Produkt zyklisiert,

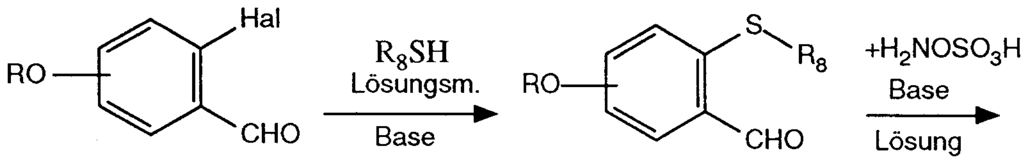


I,

II III

wobei Hal Halogen und $R_8$ $C_1$-$C_{30}$-Alkyl, $C_3$-$C_7$-Cycloalkyl oder Benzyl bedeuten.

9. Verfahren gemäss Anspruch 8, dadurch gekennzeichnet, dass man den Thioether der Formel III mit Hydroxylamin-O-sulfonsäure in Lösung umsetzt und durch Zugabe einer Base zum erwünschten Produkt zyklisiert.

10. Verfahren zur Herstellung einer Verbindung der Formel Ib

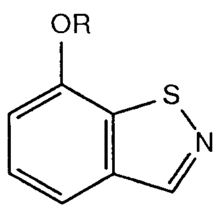

(Ib),

gemäss Anspruch 8, dadurch gekennzeichnet, dass man einen Benzaldehyd der Formel IV mit Benzylmercaptan in Lösung, in Gegenwart einer Base zum Thioether der Formel V umsetzt und letzteren, mit oder ohne Isolierung, mit Hydroxylamin-O-sulfonsäure in Lösung umsetzt und zum erwünschten Produkt zyklisiert,

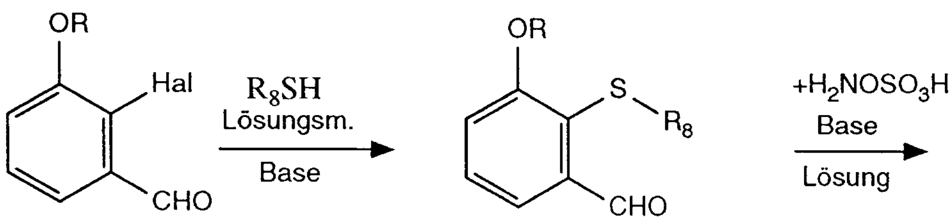


Ib,

IV V

wobei Hal Halogen und $R_8$ Benzyl bedeuten.

11. Verfahren gemäss Anspruch 10, dadurch gekennzeichnet, dass man den Thioether der Formel V mit Hydroxylamin-O-sulfonsäure in Lösung umsetzt und zum erwünschten Produkt zyklisiert.

12. Verfahren gemäss Anspruch 11, dadurch gekennzeichnet, dass man das Zwischenprodukt der Formel V mit Hydroxylamin-O-sulfonsäure bei pH = 4-6 umsetzt und die Zyklisierung durch anschliessende Base-Zugabe durchführt.

13. Verfahren zur Herstellung einer Verbindung der Formel I, worin R die in Anspruch 1 gegebenen Bedeutungen hat, mit Ausnahme der Verbindungen 4-Methoxy-benzisothiazol und 7-Methoxy-benzisothiazol, dadurch gekennzeichnet, dass man ein Benzaldehyd-Derivat der Formel VI, worin R und $R_8$ die in Anspruch 8 gegebenen Bedeutungen haben, mit Hydroxylamin oder einem Salz davon zum entsprechenden Oxim der Formel VII umsetzt und dieses mit einer starken wasserbindenden Säure zyklisiert.

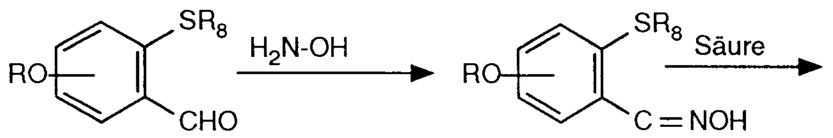


I

VI VII

14. Verfahren gemäss Anspruch 13, dadurch gekennzeichnet,dass man als starke Säure Polyphosphorsäure oder Phosphorpentoxid in Methansulfonsäure verwendet.

15. Verfahren gemäss Anspruch 14, dadurch gekennzeichnet,dass man die Reaktion in einem Gemisch von 1-20 Gewichtsprozent Phosphorpentoxid in Methansulfonsäure bei -10° bis +100°C durchführt.

16. Verfahren zur Herstellung einer Verbindung der Formel I, worin R die in Anspruch 1 gegebenen Bedeutungen hat, mit Ausnahme der Verbindungen 4-Methoxy-benzisothiazol und 7-Methoxy-benzisothiazol, dadurch gekennzeichnet, dass ein phenolartiges Hydroxy-benzisothiazol der Formel VIII

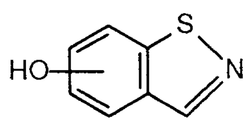

(VIII)

mit einem Reagenz R-X umgesetzt wird, in dem R die für Formel I gegebene Bedeutung hat und X Halogen, Tosylat, oder Mesylat bedeutet.

17. Verwendung eines Mittels zur Bekämpfung von pflanzenschädigende Nematoden, dadurch gekennzeichnet, dass dieses neben einem inerten Trägermaterial mindestens eine Verbindung der Formel I

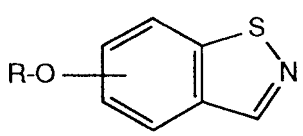

(I),

enthält, worin die Gruppe RO- in einer der Positionen des Benzolrings steht und R einen reinen oder halogenierten Kohlenwasserstoffrest bedeutet, der aliphatisch oder cycloaliphatisch, gesättigt oder ungesättigt ist.

18. Verwendung eines Mittels gemäss Anspruch 17, dadurch gekennzeichnet, dass es sich bei den Nematoden uni die Gattung der Meloidogyne und der Heterodera handelt.

**19.** Verfahren zur Bekämpfung oder Verhütung eines Befalls von Kulturpflanzen durch Nematoden, dadurch gekennzeichnet,dass man eine Verbindung der Formel I gemäss Anspruch 17 oder ein Mittel, dass mindestens eine Verbindung der Formel I gemäss Anspruch 17 enthält, auf die Pflanze, auf Pflanzenteile oder auf den Nährboden für die Pflanze appliziert.

**20.** Verfahren gemäss Anspruch 19, dadurch gekennzeichnet, dass man eine Verbindung gemäss Anspruch 4 appliziert.

**21.** Verfahren gemäss Anspruch 19, dadurch gekennzeichnet, dass man eine Verbindung gemäss Anspruch 5 appliziert.

**22.** 6-Hydroxy-benzisothiazol und 7-Hydroxy-benzisothiazol.

**Claims**

**1.** A pesticide composition for controlling or preventing attack of plants by nematodes, which comprises, besides an inert carrier material, at least one compound of the formula I

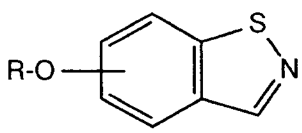

(I),

in which the group RO- is in one of the positions of the benzene ring and R is a pure or halogenated hydrocarbon radical which is aliphatic or cycloaliphatic and saturated or unsaturated, with the exception of the compounds 4-methoxybenzisothiazole and 7-methoxybenzisothiazole.

**2.** A composition according to claim 1, wherein R is a halogenated or unhalogenated hydrocarbon radical having up to 4 C atoms.

**3.** A composition according to claim 2, which comprises, as active component, a compound of the formula I'

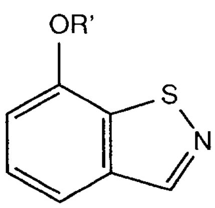

(I')

in which R' is a halogenated or unhalogenated hydrocarbon radical having 1 to 3 C atoms.

**4.** A composition according to claim 1, which comprises, as active component, a compound selected from
7-difluoromethoxybenzisothiazole,
7-allyloxybenzisothiazole
7-propargyloxybenzisothiazole,
5-methoxybenzisothiazole, and
6-methoxybenzisothiazole.

**5.** A composition according to claim 1, which comprises, as active component, a compound selected from amongst 6-ethoxybenzisothiazole and 6-allyloxybenzisothiazole.

**6.** Hydrocarbyloxybenzisothiazole of the formula I"

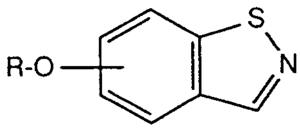

(I"),

in which the group RO- is in one of the positions of the benzene ring and R is a pure or halogenated hydrocarbon radical which is aliphatic or cycloaliphatic and saturated or unsaturated, with the proviso that a methoxy group in the 4- or 7-position is halogenated.

**7.** 6-Ethoxybenzisothiazole and 6-allyloxybenzisothiazole.

**8.** A process for the preparation of a compounds of the formula I

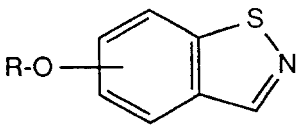

(I),

in which R is as defined in claim 1, with the exception of the compounds 4-methoxybenzisothiazole and 7-methoxybenzisothiazole , which comprises reacting a substituted halobenzaldehyde of the formula II with a thiol $R_8SH$ in solution and in the presence of a base to give the thioether of the formula III, and reacting the latter, with or without isolation, with hydroxylamine-O-sulfonic acid in solution, and cyclising the product to the desired compound,

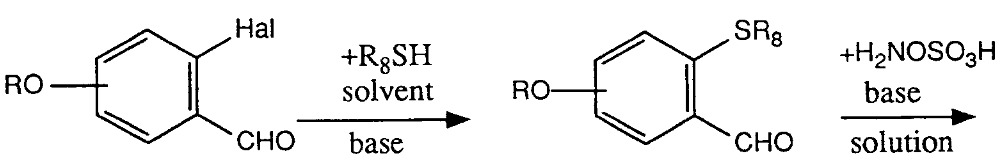


II

III

in which Hal is halogen and $R_8$ is $C_1$-$C_{30}$alkyl, $C_3$-$C_7$cycloalkyl or benzyl.

**9.** A process according to claim 8, which comprises reacting the thioether of the formula III with hydroxylamine-O-sulfonic acid in solution and cyclising the product by addition of a base to give the desired compound.

**10.** A process for the preparation of a compound of the formula Ib

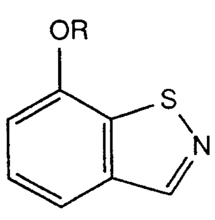

(Ib),

according to claim 8, which comprises reacting a benzaldehyde of the formula IV with benzylmercaptan in solution, reacting the product in the presence of a base to give the thioether of the formula V, reacting the latter, with or without

isolation, with hydroxylamine-O-sulfonic acid in solution, and cyclising the product to give the desired compound,

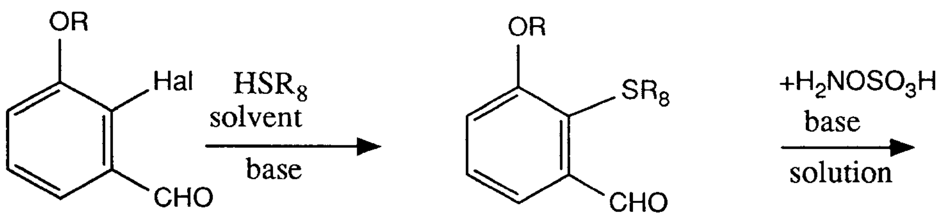


IV V

in which Hal is halogen and $R_8$ is benzyl.

**11.** A process according to claim 10, which comprises reacting the thioether of the formula V with hydroxylamine-O-sulfonic acid in solution and cyclising the product to give the desired compound.

**12.** A process according to claim 11, which comprises reacting the intermediate of the formula V with hydroxylamine-O-sulfonic acid at pH = 4-6 and carrying out the cyclisation by subsequently adding a base.

**13.** A process for the preparation of a compound of the formula I in which R is as defined in claim 1, with the exception of the compounds 4-methoxybenzisothiazole and 7-methoxybenzisothiazole, which comprises reacting a benzaldehyde derivative of the formula VI in which R and $R_8$ are as defined in claim 8 with hydroxylamine or a salt thereof to give the corresponding oxime of the formula VII and cyclising the latter with a strong water-binding acid.

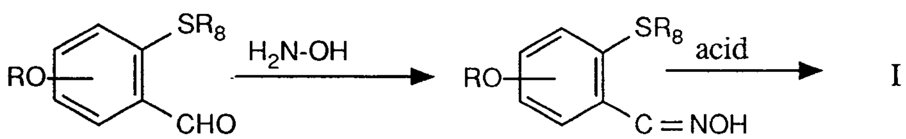


VI VII

**14.** A process according to claim 13, characterised in that the strong acid used is polyphosphoric acid or phosphorus pentoxide in methanesulfonic acid.

**15.** A process according to claim 14, which comprises carrying out the reaction at -10° to +100°C in a mixture of 1-20 per cent by weight of phosphorus pentoxide in methanesulfonic acid.

**16.** A process for the preparation of a compound of the formula I in which R is as defined in claim 1, with the exception of the compounds 4-methoxybenzisothiazole and 7-methoxybenzisothiazole, which comprises reacting a phenol-type hydroxybenzisothiazole of the formula VIII

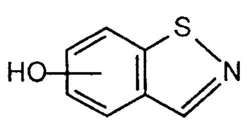

(VIII)

with a reagent R-X in which R is as defined for formula I and X is halogen, tosylate or mesylate.

**17.** The use of a composition for controlling plant-injurious nematodes, which comprises, besides an inert carrier material, at least one compound of the formula I

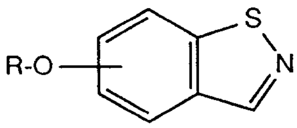

(I),

in which the group RO- is in one of the positions of the benzene ring and R is a pure or halogenated hydrocarbon radical which is aliphatic or cycloaliphatic and saturated or unsaturated.

**18.** The use of a composition according to claim 17, wherein the nematodes are from the genus Meloidogyne and Heterodera.

**19.** A method for controlling or preventing attack of crop plants by nematodes, which comprises applying a compound of the formula I according to claim 17 or a composition comprising at least one compound of the formula I according to claim 17 to the plant, to parts of the plant or to the locus of the plant.

**20.** A method according to claim 19, which comprises applying a compound according to claim 4.

**21.** A method according to claim 19, which comprises applying a compound according to claim 5.

**22.** 6-Hydroxybenzisothiazole and 7-hydroxybenzisothiazole.

**Revendications**

**1.** Agent pesticide pour maîtriser ou prévenir une infestation des végétaux par des nématodes, caractérisé en ce qu'il contient, outre un support inerte, au moins un composé de formule (I) :

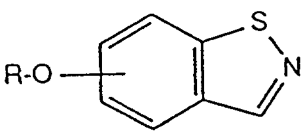

(I),

dans laquelle le groupe RO- se trouve dans l'une des positions du noyau benzénique, et R est un radical hydrocarboné pur ou halogéné, qui est aliphatique ou cycloaliphatique, saturé ou insaturé, à l'exception des composés 4-méthoxybenzisothiazole et 7-méthoxybenzisothiazole.

**2.** Agent selon la revendication 1, caractérisé en ce que R est un radical hydrocarboné éventuellement halogéné ayant jusqu'à 4 atomes de carbone.

**3.** Agent selon la revendication 2, caractérisé en ce qu'il contient comme matière active un composé de formule (I') :

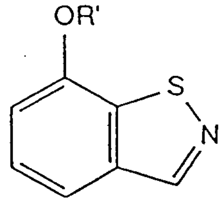

(I')

dans laquelle R' est un radical hydrocarboné éventuellement halogéné ayant de 1 à 3 atomes de carbone.

**4.** Agent selon la revendication 1, caractérisé en ce qu'il contient comme matière active un composé choisi parmi le 7-difluorométhoxybenzisothiazole, le 7-allyloxybenzisothiazole, le 7-propargyloxybenzisothiazole, le 5-méthoxybenzisothiazole et le 6-méthoxybenzisothiazole.

**5.** Agent selon la revendication 1, caractérisé en ce qu'il contient comme matière active un composé choisi parmi le 6-éthoxybenzisothiazole et le 6-allyloxybenzisothiazole.

**6.** Hydrocarbyloxy-benzisothiazole de formule (I") :

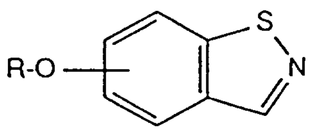

(I"),

dans laquelle le groupe RO- se trouve dans l'une des positions du noyau benzénique, et R est un radical hydrocarboné pur ou halogéné, qui est aliphatique ou cycloaliphatique, saturé ou insaturé, à la condition qu'un groupe méthoxy se trouvant en position 4 ou 7 soit halogéné.

**7.** 6-éthoxybenzisothiazole et 6-allyloxybenzisothiazole.

**8.** Procédé pour préparer un composé de formule (I) :

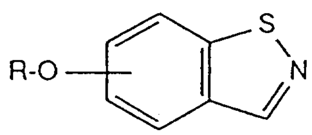

(I),

dans laquelle R a les significations données dans la revendication 1, à l'exception des composés 4-méthoxybenzisothiazole et 7-méthoxybenzisothiazole, caractérisé en ce qu'on fait réagir un halogénobenzaldéhyde substitué de formule (II) avec un thiol $R_8SH$ en solution, en présence d'une base, pour obtenir le thioéther de formule (III), et qu'on fait réagir ce dernier, avec ou sans isolement, avec de l'acide hydroxylamine-O-sulfonique en solution, et qu'on le cyclise pour obtenir le produit recherché

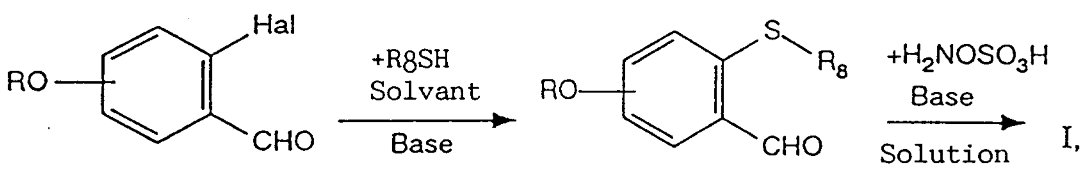


II III

où Hal est un halogène et $R_8$ est un radical alkyle en $C_1$-$C_{30}$, cycloalkyle en $C_3$-$C_7$ ou benzyle.

**9.** Procédé selon la revendication 8, caractérisé en ce qu'on fait réagir en solution le thioéther de formule (III) avec de l'acide hydroxylamine-O-sulfonique, et qu'on le cyclise par addition d'une base pour obtenir le produit recherché.

**10.** Procédé pour préparer un composé de formule (Ib) :

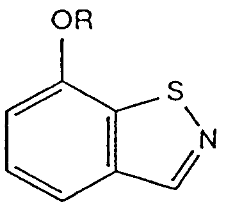

(Ib),

selon la revendication 8, caractérisé en ce qu'on fait réagir en solution, en présence d'une base, un benzaldéhyde de formule (IV) avec du benzylmercaptan pour obtenir le thioéther de formule (V), et on fait réagir ce dernier, avec ou sans isolement, avec de l'acide hydroxylamine-O-sulfonique en solution, et on le cyclise pour obtenir le produit recherché :

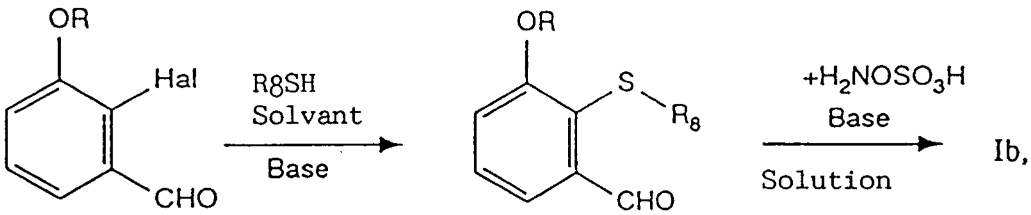


IV V

où Hal est un halogène et $R_8$ est le radical benzyle.

**11.** Procédé selon la revendication 10, caractérisé en ce qu'on fait réagir en solution le thioéther de formule (V) avec de l'acide hydroxylamine-O-sulfonique, et qu'on le cyclise en le produit recherché.

**12.** Procédé selon la revendication 11, caractérisé en ce qu'on fait réagir le produit intermédiaire de formule (V) avec de l'acide hydroxylamine-O-sulfonique à pH 4-6, et qu'on procède à la cyclisation par l'addition ultérieure d'une base.

**13.** Procédé pour préparer un composé de formule (I), dans laquelle R a les significations données dans la revendication 1, à l'exception des composés 4-méthoxybenzisothiazole et 7-méthoxybenzisothiazole, caractérisé en ce qu'on fait réagir un dérivé du benzaldéhyde de formule (VI), dans laquelle R et $R_8$ ont les significations données dans la revendication 8, avec de l'hydroxylamine ou un sel de cette dernière, pour obtenir l'oxime correspondant de formule (VII), et qu'on cyclise ce dernier avec un acide fort qui fixe l'eau.

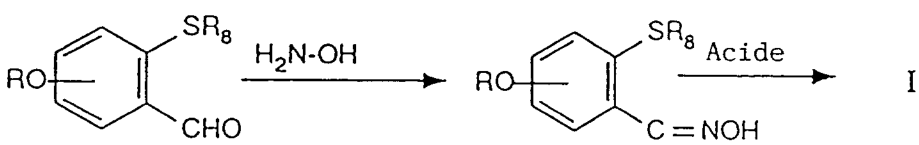


VI VII

**14.** Procédé selon la revendication 13, caractérisé en ce qu'on utilise comme acide fort de l'acide polyphosphorique ou du pentoxyde de phosphore dans de l'acide méthanesulfonique.

**15.** Procédé selon la revendication 14, caractérisé en ce qu'on met en oeuvre la réaction dans un mélange de 1 à 20 % en poids de pentoxyde de phosphore dans de l'acide méthanesulfonique à une température de -10 à +100°C.

**16.** Procédé pour préparer un composé de formule (I) dans laquelle R a les significations données dans la revendication 1, à l'exception des composés 4-méthoxybenzisothiazole et 7-méthoxybenzisothiazole, caractérisé en ce qu'on fait réagir un hydroxybenzisothiazole de type phénol, de formule (VIII) :

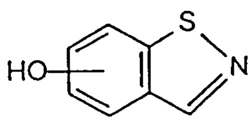

(VIII)

avec un réactif R-X, où R a les significations données pour la formule (I) et X est un halogène, un tosylate ou un mésylate.

**17.** Utilisation d'un agent destiné à maîtriser les nématodes ravageurs des végétaux, caractérisé en ce qu'il contient, outre un support inerte, au moins un composé de formule (I) :

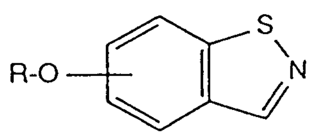

(I),

dans laquelle le groupe RO- se trouve dans l'une des positions du noyau benzénique, et R est un radical hydrocarboné pur ou halogéné, qui est aliphatique ou cycloaliphatique, saturé ou insaturé.

**18.** Utilisation d'un agent selon la revendication 17, caractérisé en ce que, pour ce qui est des nématodes, il s'agit des espèces Meloidogyne et Heterodera.

**19.** Procédé pour maîtriser ou prévenir une infestation de plantes de culture par des nématodes, caractérisé en ce qu'on applique sur les plantes, sur des parties de plantes, ou sur le sol nutritif destiné aux plantes, un composé de formule (I) selon la revendication 17 ou un agent contenant au moins un composé de formule (I) selon la revendication 17.

**20.** Procédé selon la revendication 19, caractérisé en ce qu'on applique un composé selon la revendication 4.

**21.** Procédé selon la revendication 19, caractérisé en ce qu'on applique un composé selon la revendication 5.

**22.** 6-Hydroxybenzisothiazole et 7-Hydroxybenzisothiazole.